# EUROPEAN PATENT APPLICATION

(11) **EP 3 056 232 A1**
(43) Date of publication of application: **17.08.2016**
(21) Application number: 16154869.8
(22) Date of filing: 09.02.2016
(51) Int. Cl.: A61M 1/14, G01N 31/00, G01N 21/76, G01N 33/62, G01N 33/18

(54) **METHOD AND APPARATUS FOR MEASURING UREA CONCENTRATION**

(30) Priority: 10.02.2015 JP 2015024181
(71) Applicant: Ricoh Company Ltd., Tokyo 143-8555 (JP); Kake Educational Institution, Okayama 700-0005 (JP)
(72) Inventor: HASHIGUCHI, Masafumi, Ohta-ku, Tokyo 143-8555 (JP); NAKAGAWA, Masuo, Okayama-shi, Okayama 700-0005 (JP); SHINHAMA, Kousuke, Okayama-shi, Okayama 700-0005 (JP); FUKUSHIMA, Yasuhide, Ohta-ku, Tokyo 143-8555 (JP)
(74) Representative: Lamb, Martin John Carstairs

(57) **Abstract**

Provided is a method and apparatus for measuring a urea concentration. The method includes adding a first reagent comprising a hypohalite ion, preferably a hypofluorite ion, a hypochlorite ion, a hypobromite ion, or/and a hypoiodite ion, and a second reagent comprising a xanthene dye, preferably rhodamine B, fluorescein, dibromofluorescein, dichlorofluorescein or/and a rare earth fluorescent complex to a sample solution including urea to be allowed to undergo a reaction, and measuring light intensity of chemiluminescence produced through the reaction to determine the urea concentration, preferably in real time, in the sample solution comprising spend dialysate during hemodialysis. The apparatus for measuring a urea concentration, the apparatus comprising a second-reagent adding unit comprising a second reagent (21) tank, a pump (15) and a liquid feeding path (10) configured to add a second reagent comprising a xanthene dye or a rare earth fluorescent complex to a sample solution comprising urea to be mixed together to form a mixed solution (21); a first-reagent adding unit comprising a first reagent (22) tank, a pump (16) and a liquid feeding path (11) configured to add a first reagent comprising a hypohalite ion to the mixed solution to be allowed to undergo a reaction in a reaction cell (2); and a measuring unit comprising an optical detector (5) and a measuring device (7) configured to measure light intensity of chemiluminescence produced through the reaction to determine the urea concentration.

## Description

### BACKGROUND OF THE INVENTION

### Field of the Invention

The present invention relates to methods for measuring a urea concentration and apparatuses for measuring urea concentration.

### Description of the Related Art

Conventionally, the following methods have been known as a method for measuring a urea concentration in a sample solution including urea: a colorimetric method in which a reagent that reacts with urea to develop color is used to compare the developed color with the standard color of the reagent to thereby determine the urea concentration; an enzyme thermistor method in which urease that is an enzyme specifically acting on urea is immobilized onto glass beads and heat of reaction generated during a hydrolysis reaction of urea is measured to thereby determine the urea concentration; and a chemiluminescence method in which a reagent that reacts with urea to produce chemiluminescence (e.g., hypohalite) is used to determine the urea concentration based on light intensity of the chemiluminescence.

Among them, the chemiluminescence method can be used in dialysis therapy as a unit enabling the timing to complete dialysis to be informed because the urea concentration in the sample solution can be measured in real time.

However, the light intensity of the chemiluminescence is so weak that an expensive, large-sized photomultiplier tube has been used to sensitize the light intensity of the chemiluminescence in the case of measuring the urea concentration (10 mg/dL to 30 mg/dL) in spent dialysate during dialysis (see, for example, Japanese Patent Application Laid-Open (JP-A) No. 2008-14910).

There have been many attempts to enhance the light intensity of the chemiluminescence, for example, by adjusting a concentration of a reagent or a mixing speed of a sample solution and the reagent, but sufficient light intensity of the chemiluminescence has not been achieved. Furthermore, there is a problem that a complex or large-scale system is needed because a concentration and a flow rate of the reagent are required to be controlled to enhance the light intensity of the chemiluminescence.

### SUMMARY OF THE INVENTION

A object of the present invention is provide a method for measuring a urea concentration that is capable of measuring the urea concentration in a sample solution in a rapid, highly precise, and highly sensitive manner and that is capable of realizing a downsized apparatus.

As a means for solving the above problems, a method for measuring a urea concentration of the present invention includes adding a first reagent and a second reagent to a sample solution including urea to be allowed to undergo a reaction, and measuring light intensity of chemiluminescence produced through the reaction to determine the urea concentration, the first reagent including a hypohalite ion, the second reagent including a xanthene dye or a rare earth fluorescent complex.

The present invention can provide a method for measuring a urea concentration that is capable of measuring the urea concentration in a sample solution in a rapid, highly precise, and highly sensitive manner and that is capable of realizing a downsized apparatus.

### BRIEF DESCRIPTION OF THE DRAWINGS

FIG. 1 is a schematic diagram illustrating one exemplary apparatus for measuring a urea concentration according to the present invention;
FIG. 2 is a schematic diagram illustrating another exemplary apparatus for measuring a urea concentration according to the present invention;
FIG. 3 is a graph illustrating a relationship between urea concentrations and output voltages in Example 1;
FIG. 4 is a graph illustrating a sensitizing effect of Rhodamine B in Example 1;
FIG. 5 is a graph illustrating a relationship between urea concentrations and output voltages in Example 2;
FIG. 6 is a graph illustrating a sensitizing effect of fluorescein (FS) in Example 2;
FIG. 7 is a graph illustrating a relationship between urea concentrations and output voltages in Example 3;
FIG. 8 is a graph illustrating a sensitizing effect of dibromofluorescein (DBFS) in Example 3;
FIG. 9 is a graph illustrating a relationship between urea concentrations and output voltages in Example 4;
FIG. 10 is a graph illustrating a sensitizing effect of dichlorofluorescein (DCFS) in Example 4;
FIG. 11 is a graph illustrating a change of a wavelength property by the addition of Rhodamine B; and
FIG. 12 is a graph illustrating a change of a wavelength property by the addition of fluorescein (FS).

### DETAILED DESCRIPTION OF THE INVENTION

### (Method for measuring urea concentration)

A method for measuring a urea concentration of the present invention includes adding a first reagent and a second reagent to a sample solution including urea to be allowed to undergo a reaction, and measuring light intensity of chemiluminescence produced through the reaction to determine the urea concentration, the first reagent including a hypohalite ion, the second reagent including a xanthene dye or a rare earth fluorescent complex.

In the present invention, the light intensity of the chemiluminescence produced through a reaction between the sample solution including urea and the first reagent is greatly sensitized by the action of the second reagent. As a result, an inexpensive, small-sized semiconductor optical sensor can be used as an optical detector instead of an expensive, large-sized photomultiplier tube. Thus, a downsized apparatus capable of measuring the urea concentration in the sample solution in a highly sensitive and efficient manner even with a simple configuration can be realized.

The first reagent and the second reagent may be added to the sample solution including urea simultaneously. However, it is preferable that the second reagent be added to and mixed with the sample solution including urea to form a mixed solution, and the first reagent be mixed with the mixed solution to be allowed to undergo the reaction. This makes it possible to prevent a decrease of the light intensity of the chemiluminescence due to a reaction of the first reagent with the second reagent prior to with the sample solution, resulting in stable chemiluminescence.

### <Sample solution including urea>

The sample solution including urea is not particularly limited and may be appropriately selected depending on the intended purpose, as long as the sample solution includes urea that is an object to be measured. Examples of the sample solution include spent dialysate during hemodialysis, polluted water from an excreta or waste water disposal treatment plant in a stock farm, and plasma in blood.

A urea nitrogen level in the spent dialysate during hemodialysis is about 28% relative to a urea nitrogen level in blood, and is usually 2 mg/dL to 28 mg/dL.

### <First reagent>

The first reagent is a solution including a hypohalite ion.

Examples of the hypohalite ion include a hypofluorite ion (FO⁻), a hypochlorite ion (ClO⁻), a hypobromite ion (BrO⁻), and a hypoiodite ion (IO⁻). Among them, the hypobromite ion (BrO⁻) is particularly preferable in terms of producing chemiluminescence.

An aqueous sodium hypobromite solution serving as the first reagent can be prepared by dissolving NaOH, NaBr, and NaClO in water.

Example of a hypohalite salt includes sodium hypobromite.

The first reagent is preferably added to the sample solution including urea so as to give a concentration of 50 mmol/L to 300 mmol/L.

### <Second reagent>

The second reagent can be added to the sample solution including urea to thereby control the maximum emission wavelength and adjust an emission wavelength in accordance with sensitivity of the optical detector. This enables efficient light receiving and highly sensitive measurement.

The second reagent includes a xanthene dye or a rare earth fluorescent complex.

Examples of the xanthene dye include Rhodamine B, fluorescein, dichlorofluorescein, dibromofluorescein, and diiodofluorescein.

Example of the rare earth fluorescent complex includes Eu(β-NTA)₃(TOPO)₂.

Among them, from the viewpoint of a sensitizing effect, particularly preferable are the Rhodamine B, the fluorescein, the dibromofluorescein, and the dichlorofluorescein.

The second reagent is preferably added to the sample solution including urea so as to give a concentration of 0.2 mmol/L to 10 mmol/L.

In the case of using the Rhodamine B as the second reagent, the Rhodamine B is added to the sample solution including urea in a concentration of 0.2 mmol/L or higher.

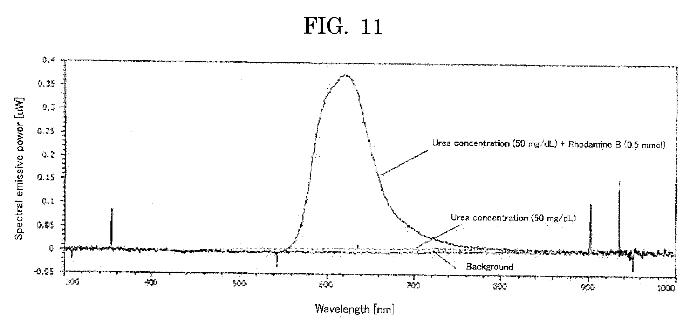

The use of the Rhodamine B having the concentration of 0.2 mmol/L or higher as the second reagent can result in light intensity localized at a wavelength of about 620 nm (see, FIG. 11).

In the case of using the fluorescein as the second reagent, the fluorescein is added to the sample solution including urea in a concentration of 2 mmol/L or higher.

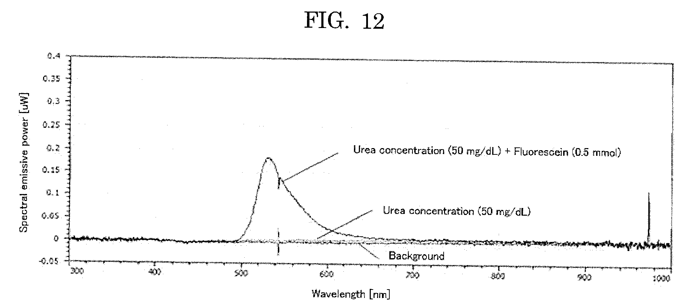

The use of the fluorescein having the concentration of 2 mmol/L or higher as the second reagent can result in light intensity localized at a wavelength of about 520 nm (see, FIG. 12).

In the case of using the dibromofluorescein as the second reagent, the dibromofluorescein is added to the sample solution including urea in a concentration of 2 mmol/L or higher.

The use of the dibromofluorescein having the concentration of 2 mmol/L or higher as the second reagent can result in light intensity localized at a wavelength of about 520 nm.

In the case of using the dichlorofluorescein as the second reagent, the dichlorofluorescein is added to the sample solution including urea in a concentration of 2 mmol/L or higher.

The use of the dichlorofluorescein having the concentration of 2 mmol/L or higher as the second reagent can result in light intensity localized at a wavelength of about 520 nm.

### <Chemiluminescence>

The chemiluminescence (CL) is produced when an excited nitrogen (N₂*) produced during a reaction between the urea and the hypobromite ion serving as the first reagent returns to the ground state, as illustrated by the following formulae:

(NH₂)₂CO + 2OBr⁻ → N₂* + 2Br⁻

N₂* → N₂ + h*ν*

The chemiluminescence (CL) begin to be produced from the moment the urea and the hypobromite ion serving as the first reagent are mixed. The duration of the chemiluminescence is of the order of some tens to some hundreds of milliseconds.

In the present invention, the intensity of the chemiluminescence can be greatly sensitized by the addition of the xanthene dye or rare earth fluorescent complex serving as the second reagent.

The intensity of the chemiluminescence produced is proportional to the urea concentration in the sample solution. Therefore, the chemiluminescence can be measured by amperometry with an optical detector and a measuring device to thereby determine the urea concentration.

In the present invention, the intensity of chemiluminescence is greatly sensitized by the addition of the second agent, so that an inexpensive, small-sized semiconductor optical sensor can be employed as the optical detector instead of an expensive, large-sized photomultiplier tube.

### (Apparatus for measuring urea concentration)

An apparatus for measuring a urea concentration of the present invention is an apparatus used in the method for measuring a urea concentration of the present invention. The apparatus for measuring a urea concentration includes a first-reagent adding unit, a second-reagent adding unit, and a measuring unit; preferably includes a first and second reagents adding unit; and, if necessary, further includes other units.

### <Second-reagent adding unit>

The second-reagent adding unit is a unit configured to add the second reagent including a xanthene dye or a rare earth fluorescent complex to the sample solution including urea to mix together to thereby form a mixed solution.

Example of the second-reagent adding unit includes a combination of a second reagent tank, a liquid feeding path, and a pump. Examples of the pump include a diaphragm pump and a peristaltic pump.

The xanthene dye or the rare earth fluorescent complex may be the same as those described for the method for measuring a urea concentration.

### <First-reagent adding unit>

The first-reagent adding unit is a unit configured to add the first reagent including a hypohalite ion to the mixed solution to be allowed to undergo a reaction in a reaction cell.

Example of the first-reagent adding unit includes a combination of a first reagent tank, a liquid feeding path, and a pump. Examples of the pump include a diaphragm pump and a peristaltic pump.

The material, shape, size, and structure of the reaction cell are not particularly limited and may be appropriately selected depending on the intended purpose.

The material of the reaction cell is not particularly limited and may be appropriately selected depending on the intended purpose, as long as the material is transparent and unreactive with the first reagent or the second reagent. Examples of the material include an acrylic resin, polytetrafluoroethylene, silicones such as polydimethylsiloxane; polystyrene, polyester, aromatic polyamide, nylon, polyolefin; metal such as stainless; glass, (crystalline) silicon, and quartz.

The reaction cell may be, for example, cylindrical shaped.

### <First and second reagents adding unit>

The first and second reagents adding unit is a unit configured to add the second reagent to the sample solution including urea to be mixed together to form a mixed solution, and to add the first reagent to the mixed solution to be allowed to undergo a reaction in a reaction cell.

In the present invention, the first and second reagents adding unit may be used instead of the first-reagent adding unit and the second-reagent adding unit. Thus, the first reagent can be mixed with the mixed solution that is formed by adding the second reagent to the sample solution including urea to mix together in advance, followed by allowing a reaction with each other. This makes it possible to prevent a decrease of the light intensity of the chemiluminescence due to a reaction of the first reagent with the second reagent prior to with the sample solution, resulting in stable chemiluminescence.

Example of the first and second reagents adding unit includes a combination of a second reagent tank, a liquid feeding path, and a pump. Examples of the pump include a diaphragm pump and a peristaltic pump.

### <Measuring unit>

The measuring unit is a unit configured to measure the light intensity of the chemiluminescence produced through the reaction to determine the urea concentration.

Example of the measuring unit includes a combination of an optical detector and a measuring device. In the present invention, an inexpensive, small-sized semiconductor optical sensor can be used as the optical detector instead of an expensive, large-sized photomultiplier tube.

Example of the measuring device includes a picoammeter.

### <Other units>

The other units are not particularly limited and may be appropriately selected depending on the intended purpose. Examples of the other units include a heating unit, a stirrer, and a control unit.

Example of the heating unit includes a heatblock. The above described chemical reaction depends on temperature, so that the reaction cell and adjacent thereto is preferably kept at a predetermined temperature. The predetermined temperature is preferably 20°C to 40°C.

Examples of the stirrer include a microreactor and a magnetic stirrer.

Examples of the control unit include a temperature controller and a microcomputer.

The method for measuring a urea concentration and the apparatus for measuring a urea concentration of the present invention are not particularly limited and may be used for various applications such as in a dialyzer configured to measure a urea concentration in spent dialysate during dialysis, and a water quality analyzer used in an excreta or waste water disposal treatment plant in a stock farm.

In particular, the method and the apparatus of the present invention can be particularly suitably used in a dialysis monitoring device that is capable of determining the dialysis time suitable for each patient because they can measure the urea concentration in the spent dialysate during dialysis in real time unlike a conventional, time-consuming urea examinations including collection of blood.

Specific embodiments of the method for a urea concentration and the apparatus for measuring a urea concentration of the present invention will now be described in detail referring to figures.

### <First embodiment>

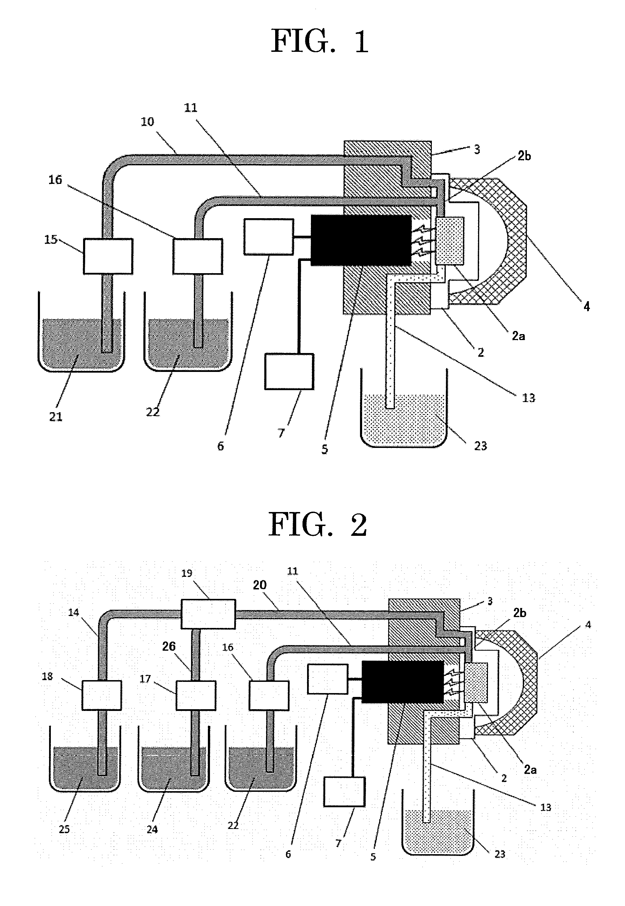

The configuration of the first embodiment of the apparatus for measuring a urea concentration according to the present invention is illustrated in FIG. 1. The method for a urea concentration using the apparatus for measuring a urea concentration illustrated in FIG. 1 will now be described.

Firstly, spent dialysate during dialysis serving as the sample solution including urea is mixed with a xanthene dye solution or a rare earth fluorescent complex serving as the second reagent to form a mixed solution 21 in advance.

The xanthene dye may be Rhodamine B, fluorescein, dibromofluorescein, or dichlorofluorescein.

The rare earth fluorescent complex may be Eu(β-NTA)₃(TOPO)₂.

The second reagent is preferably contained in the sample solution including urea in a concentration of 0.2 mmol/L to 10 mmol/L.

Next, the mixed solution 21 and a solution including a hypohalite ion 22 serving as the first reagent are respectively fed through liquid feeding paths 10 and 11 using pumps 15 and 16, and mixed with each other at a light-emitting portion 2a or a portion located immediately before the light-emitting portion 2b in a reaction cell 2 to be allowed to undergo a reaction to produce chemiluminescence at the light-emitting portion 2a.

The solution including a hypohalite ion may be a solution including a hypofluorite ion (FO⁻), a hypochlorite ion (ClO⁻), a hypobromite ion (BrO⁻), or a hypoiodite ion (IO⁻).

The first reagent is preferably contained in the sample solution including urea in a concentration of 50 mmol/L to 300 mmol/L.

The reaction cell may be a cylindrical reaction cell made of polytetrafluoroethylene with a glass window or an acrylic resin.

Then, the chemiluminescence produced in the reaction cell 2 is detected at an optical detector 5 disposed to confront the reaction cell 2, followed by measuring the urea concentration at a measuring device 7.

The optical detector 5 may be a semiconductor optical sensor. In the present invention, the light intensity of the chemiluminescence can be greatly enhanced, so that an expensive, large-sized photomultiplier tube does not have to be used.

The measuring device 7 may be a picoammeter or a current amplifier circuit.

In FIG. 1, the reference numeral 3 denotes a heating unit, the reference numeral 6 denotes a power source, the reference numeral 13 denotes a liquid feeding path, and the reference numeral 23 denotes a solution that has been measured.

The heating unit 3 may be a heatblock.

In addition, chemiluminescence emitted to directions other than a direction towards the optical detector 5 is preferably collected into the optical detector 5 by a reflector 4. However, an apparatus having a simple configuration without the reflector 4 may be used, as long as sufficient light intensity can be achieved.

The reflector 4 may be various reflecting plates, mirror reflectors, or barium sulfate reflectors.

### <Second embodiment>

Next, the configuration of the second embodiment of the apparatus for measuring a urea concentration that is capable of measuring in real time the urea concentration varying over time according to the present invention is illustrated in FIG. 2.

In the apparatus for measuring a urea concentration illustrated in FIG. 2, an aqueous urea solution 24 serving as the sample solution including urea and a xanthene dye or rare earth fluorescent complex solution 25 serving as the second reagent are respectively fed through liquid feeding paths 26 and 14 using pumps 17 and 18, and passed through a stirrer 19 to mixed together to form a mixed solution. The mixed solution is fed through a liquid feeding path 20 into a reaction cell 2.

The xanthene dye or the rare earth fluorescent complex solution may be the same as those described for the first embodiment.

The stirrer 19 may be a microreactor or a magnetic stirrer.

On the other hand, the solution including a hypobromite ion 22 serving as the first reagent is fed through a liquid feeding path 11 using a pump 16 into the reaction cell 2.

The mixed solution that is a mixture of the aqueous urea solution 24 and the second reagent 25 is mixed with the first reagent 22 at a light-emitting portion 2a or a portion located immediately before the light-emitting portion 2b in the reaction cell 2 to be allowed to undergo a reaction to produce chemiluminescence.

The chemiluminescence produced therein is detected at an optical detector 5 disposed to confront the reaction cell 2, followed by measuring with a measuring device 7.

The optical detector 5 may be a semiconductor optical sensor. In the present invention, the light intensity of the chemiluminescence can be greatly enhanced, so that an expensive, large-sized photomultiplier tube does not have to be used.

The measuring device 7 may be a picoammeter or a current amplifier circuit.

In FIG. 2, the reference numeral 3 denotes a heating unit, the reference numeral 6 denotes a power source, the reference numeral 13 denotes a liquid feeding path, and the reference numeral 23 denotes a sample solution that has been measured.

The heating unit 3 may be a heatblock.

In addition, chemiluminescence emitted to directions other than a direction towards the optical detector 5 is preferably collected into the optical detector 5 by a reflector 4. However, an apparatus having a simple configuration without the reflector 4 may be used, as long as sufficient light intensity can be achieved.

The reflector 4 may be various reflecting plates, mirror reflectors, or barium sulfate reflectors.

### Examples

Examples of the present invention will now be described, but the present invention is not limited thereto in any way.

### (Example 1)

The apparatus for measuring a urea concentration illustrated in FIG. 1 was used to mix an aqueous urea solution (concentration: 5 mg/dL, manufactured by KANTO CHEMICAL CO., INC., Grade G) serving as the sample solution, an aqueous sodium hypobromite solution (concentration: 300 mmol/L, manufactured by KANTO CHEMICAL CO., INC., Grade E) serving as the first regent, and a Rhodamine B solution (concentration: 0.2 mmol/L, manufactured by KANTO CHEMICAL CO., INC.) serving as the second reagent, followed by measuring the light intensity of chemiluminescence.

Specifically, the aqueous urea solution serving as the sample solution was mixed with the Rhodamine B solution serving as the second reagent to thereby prepare a mixed solution 21 in advance. The aqueous urea solution and the Rhodamine B solution were mixed in a volume ratio of 1:1.

Next, the mixed solution 21 was fed through a liquid feeding path 10 using a pump 15 at a flow rate of 30 mL/min, an aqueous sodium hypobromite solution 22 serving as the first regent was fed through a liquid feeding path 11 using a pump 16 at a flow rate of 30 mL/min, and the mixed solution was mixed with the aqueous sodium hypobromite solution at a light-emitting portion 2a or a portion located immediately before the light-emitting portion 2b when entering into a reaction cell 2 made of an acrylic resin to be allowed to undergo a reaction to produce chemiluminescence at the light-emitting portion 2a.

The chemiluminescence produced therein is detected at a semiconductor optical sensor (phototransistor; manufactured in-house, i.e., by Ricoh Company, Ltd.) disposed to confront the reaction cell 2 and serving as an optical detector 5, followed by measuring a urea concentration with a measuring device 7 (current amplifier circuit).

In addition, chemiluminescence emitted to directions other than a direction towards the optical detector 5 is preferably collected into the optical detector 5 by a reflector 4. However, an apparatus having a simple configuration without the reflector 4 may be used, as long as sufficient light intensity can be achieved.

In Example 1, the chemiluminescence produced through the reaction between the sample solution including urea and the first reagent was multiplicated by the action of the second reagent, so that an apparatus including an inexpensive, small-sized semiconductor optical sensor as an optical detector and having a simple configuration could be used to measure the urea concentration.

### <Evaluation of dependence on urea concentration and Rhodamine B concentration>

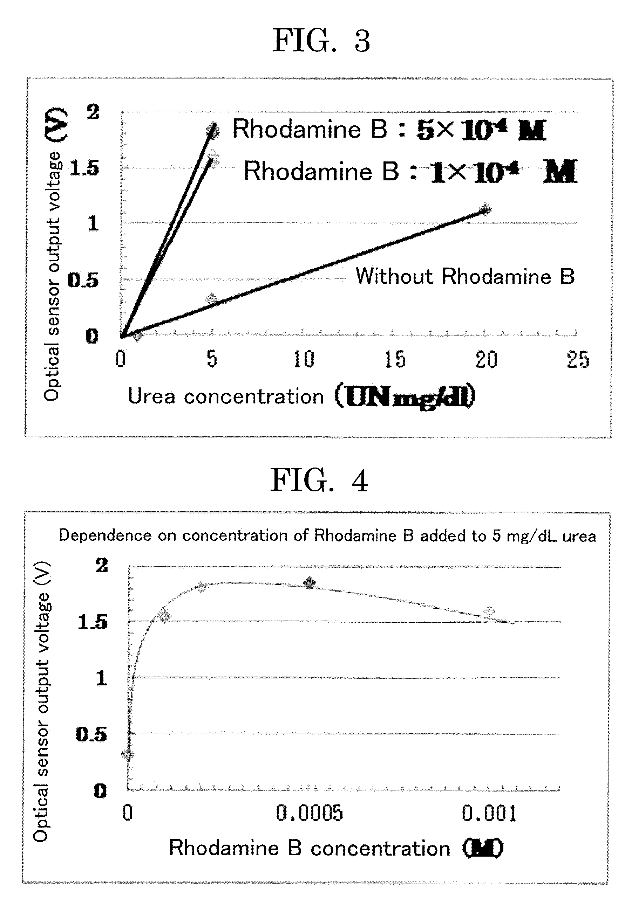

Next, light intensity localized at a wavelength of about 620 nm was measured with the photomultiplier tube (H10722-110; manufactured by Hamamatsu Photonics K.K.) in the same manner as in Example 1, except that a 0 mg/dL to 20 mg/dL aqueous urea solution was used as the sample solution, a 0.1 mmol/L to 1 mmol/L Rhodamine B solution was used as the second reagent, and an aqueous solution formed by dissolving NaOH: 0.8 mol + NaBr: 2.1 mol + NaClO: 0.3 mol in 1 L of water was used as the first reagent. Results are presented in FIGs. 3 and 4. The addition of the Rhodamine B resulted in output up to 5.8 times as high as that of without the Rhodamine B, as presented in FIG. 4.

FIG. 11 illustrates a change of a wavelength property by the addition of the Rhodamine B.

Thus, the Rhodamine B could be used as the second reagent to control the maximum emission wavelength to 620 nm (see, FIG. 11) and to adjust an emission wavelength in accordance with sensitivity of the semiconductor optical sensor serving as the optical detector. This enabled efficient light receiving and measurement.

### (Example 2)

The urea concentration was measured in the same manner as in Example 1, except that the Rhodamine B solution serving as the second reagent was changed to a 0.1 mmol/L to 5 mmol/L fluorescein solution (manufactured by KANTO CHEMICAL CO., INC.).

In Example 2, the chemiluminescence produced through the reaction between the sample solution including urea and the first reagent was multiplicated by the action of the second reagent, so that an apparatus including an inexpensive optical receiving unit and having a simple configuration could be used to measure the urea concentration.

### <Evaluation of dependence on urea concentration and fluorescein concentration>

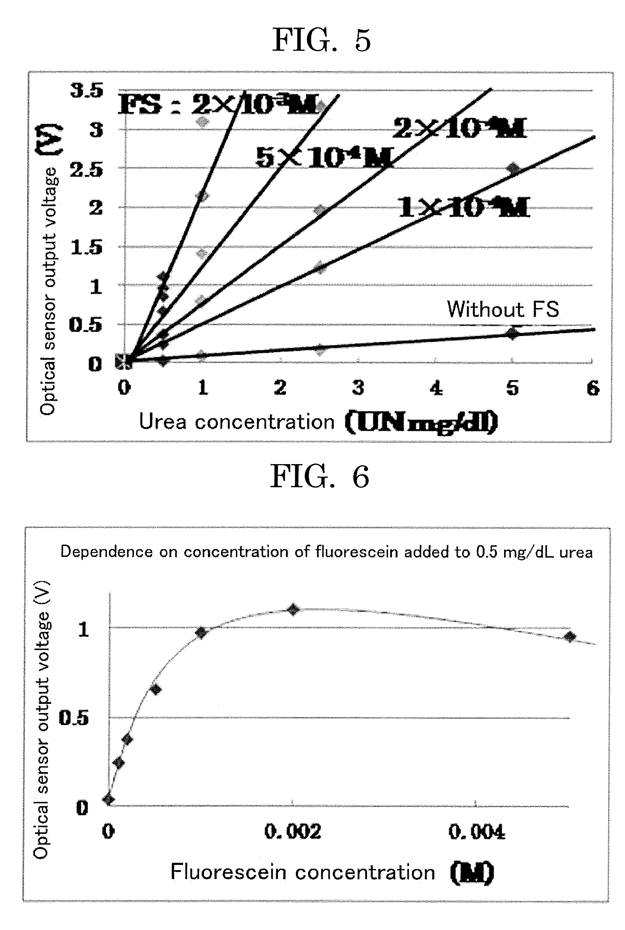

Next, light intensity localized at a wavelength of about 520 nm was measured with the photomultiplier tube (H10722-110; manufactured by Hamamatsu Photonics K.K.) in the same manner as in Example 2, except that a 0 mg/dL to 20 mg/dL aqueous urea solution was used as the sample solution, a 0.1 mmol/L to 5 mmol/L fluorescein solution was used as the second reagent, and an aqueous solution formed by dissolving NaOH: 0.8 mol + NaBr: 2.1 mol + NaClO: 0.3 mol in 1 L of water was used as the first reagent. Results are presented in FIGs. 5 and 6. The addition of the fluorescein (FS) resulted in output up to 29 times as high as that of without the fluorescein, as presented in FIG. 6.

FIG. 12 illustrates a change of a wavelength property by the addition of the fluorescein.

Thus, the fluorescein could be used as the second reagent to control the maximum emission wavelength to 520 nm (see, FIG. 12) and to adjust an emission wavelength in accordance with sensitivity of the semiconductor optical sensor serving as the optical detector. This enabled efficient light receiving and measurement.

### (Example 3)

The urea concentration was measured in the same manner as in Example 1, except that the Rhodamine B solution serving as the second reagent was changed to a 0.1 mmol/L to 2 mmol/L dibromofluorescein solution (manufactured by Tokyo Chemical Industry Co., Ltd.).

In Example 3, the chemiluminescence produced through the reaction between the sample solution including urea and the first reagent was multiplicated by the action of the second reagent, so that an apparatus including an inexpensive optical receiving unit and having a simple configuration could be used to measure the urea concentration.

### <Evaluation of dependence on urea concentration and dibromofluorescein concentration>

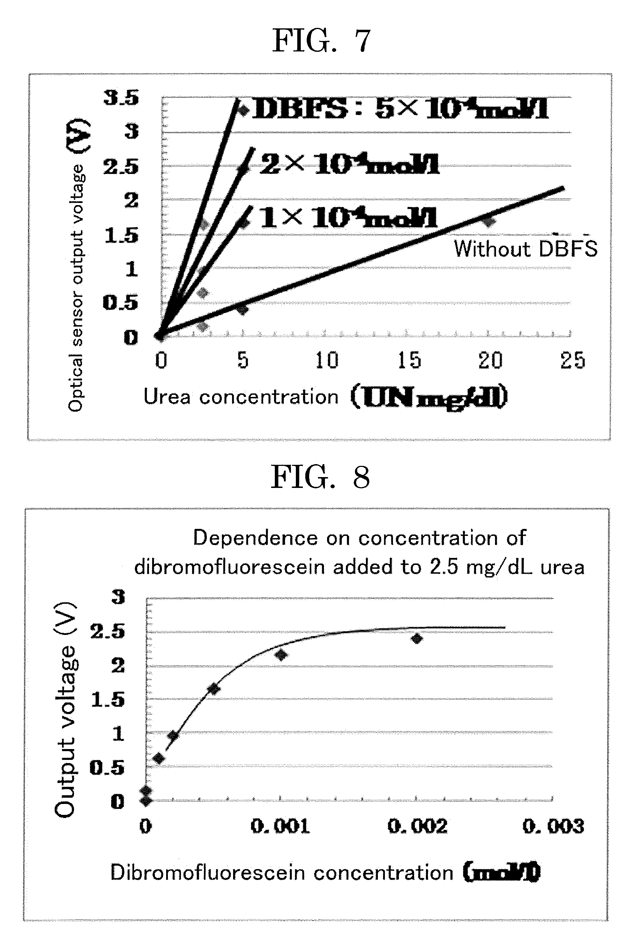

Next, light intensity localized at a wavelength of about 520 nm was measured with the photomultiplier tube (H10722-110; manufactured by Hamamatsu Photonics K.K.) in the same manner as in Example 3, except that a 0 mg/dL to 20 mg/dL aqueous urea solution was used as the sample solution, a 0.1 mmol/L to 2 mmol/L dibromofluorescein solution was used as the second reagent, and an aqueous solution formed by dissolving NaOH: 0.8 mol + NaBr: 2.1 mol + NaClO: 0.3 mol in 1 L of water was used as the first reagent. Results are presented in FIGs. 7 and 8. The addition of the dibromofluorescein (DBFS) resulted in output up to 15 times as high as that of without the dibromofluorescein, as presented in FIG. 8.

Thus, the dibromofluorescein could be used as the second reagent to control the maximum emission wavelength to 520 nm and to adjust an emission wavelength in accordance with sensitivity of the semiconductor optical sensor serving as the optical detector. This enabled efficient light receiving and measurement.

### (Example 4)

The urea concentration was measured in the same manner as in Example 1, except that the Rhodamine B solution serving as the second reagent was changed to a 0.1 mmol/L to 2 mmol/L dichlorofluorescein solution (manufactured by Tokyo Chemical Industry Co., Ltd.).

In Example 4, the chemiluminescence produced through the reaction between the sample solution including urea and the first reagent was multiplicated by the action of the second reagent, so that an apparatus including an inexpensive optical receiving unit and having a simple configuration could be used to measure the urea concentration.

### <Evaluation of dependence on urea concentration and dichlorofluorescein concentration>

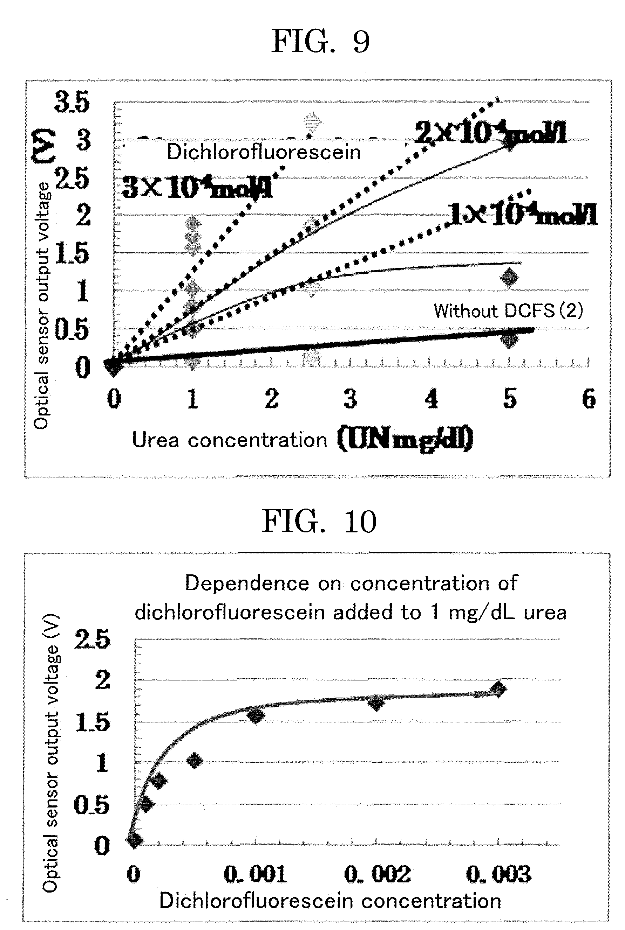

Next, light intensity localized at a wavelength of about 520 nm was measured with the photomultiplier tube (H10722-110; manufactured by Hamamatsu Photonics K.K.) in the same manner as in Example 4, except that a 0 mg/dL to 20 mg/dL aqueous urea solution was used as the sample solution, a 0.1 mmol/L to 2 mmol/L dichlorofluorescein solution was used as the second reagent, and an aqueous solution formed by dissolving NaOH: 0.8 mol + NaBr: 2.1 mol + NaClO: 0.3 mol in 1 L of water was used as the first reagent. Results are presented in FIGs. 9 and 10. The addition of the dichlorofluorescein (DCFS) resulted in output up to 20 times as high as that of without the dichlorofluorescein, as presented in FIG. 10.

Thus, the dichlorofluorescein could be used as the second reagent to control the maximum emission wavelength to 520 nm and to adjust an emission wavelength in accordance with sensitivity of the semiconductor optical sensor serving as the optical detector. This enabled efficient light receiving and measurement.

### (Example 5)

The apparatus for measuring a urea concentration illustrated in FIG. 2 was used to mix an aqueous urea solution (concentration: 5 mg/dL, manufactured by KANTO CHEMICAL CO., INC., Grade G) serving as the sample solution, an aqueous sodium hypobromite solution (concentration: 0.3 mol/L, manufactured by KANTO CHEMICAL CO., INC., Grade E) serving as the first regent, and a Rhodamine B solution (concentration: 0.2 mmol/L, manufactured by KANTO CHEMICAL CO., INC.) serving as the second reagent followed by measuring the light intensity of chemiluminescence.

Firstly, an aqueous urea solution 24 and a Rhodamine B solution 25 serving as the second reagent were respectively fed through liquid feeding paths 26 and 14 using pumps 17 and 18 and passed through a stirrer 19 to mixed together to form a mixed solution. The mixed solution is fed through a liquid feeding path 20 into a reaction cell 2 at a flow rate of 30 mL/mim. The stirrer 19 was a mixer made of polytetrafluoroethylene.

On the other hand, an aqueous sodium hypobromite solution 22 serving as the first regent was fed through a liquid feeding path 11 using a pump 16 at a flow rate of 30 mL/min and mixed with the mixed solution at a light-emitting portion 2a or a portion located immediately before the light-emitting portion 2b when entering into the reaction cell 2 made of an acrylic resin to be allowed to undergo a reaction to produce chemiluminescence at the light-emitting portion 2a.

The chemiluminescence produced therein is detected at a semiconductor optical sensor (phototransistor; manufactured in-house, i.e., by Ricoh Company, Ltd.) disposed to confront the reaction cell 2 and serving as an optical detector 5, followed by measuring a urea concentration with the measuring device 7 (current amplifier circuit).

In addition, chemiluminescence emitted to directions other than a direction towards the optical detector 5 is preferably collected into the optical detector 5 by a reflector 4. However, an apparatus having a simple configuration without the reflector 4 may be used, as long as sufficient light intensity can be achieved.

In Example 5, it is possible to prevent a decrease of the light intensity of the chemiluminescence due to a reaction of the first reagent with the second reagent prior to with the sample solution, resulting in stable chemiluminescence.

The configuration of the apparatus for measuring a urea concentration of Example 5 enables the urea concentration varying over time (such as in spent dialysate during hemodialysis) to be measured in real time and the timing to complete the dialysis to be informed.

Aspects of the present invention are as follows.
<1> A method for measuring a urea concentration, the method including: adding a first reagent and a second reagent to a sample solution including urea to be allowed to undergo a reaction, and measuring light intensity of chemiluminescence produced through the reaction to determine the urea concentration, the first reagent including a hypohalite ion, the second reagent including a xanthene dye or a rare earth fluorescent complex.
<2> The method for measuring a urea concentration according to <1>, wherein the second reagent is added to and mixed with the sample solution including urea to form a mixed solution, and the first reagent is mixed with the mixed solution to be allowed to undergo the reaction.
<3> The method for measuring a urea concentration according to <1> or <2>, wherein the xanthene dye includes at least one selected from the group consisting of Rhodamine B, fluorescein, dibromofluorescein, and dichlorofluorescein.
<4> The method for measuring a urea concentration according to any one of <1> to <3>, wherein the hypohalite ion includes at least one selected from the group consisting of a hypofluorite ion (FO-), a hypochlorite ion (ClO⁻), a hypobromite ion (BrO⁻), and a hypoiodite ion (IO⁻).
<5> The method for measuring a urea concentration according to <4>, wherein the hypohalite ion includes the hypobromite ion (BrO⁻).
<6> The method for measuring a urea concentration according to any one of <1> to <5>, wherein the sample solution including urea includes spent dialysate during hemodialysis.
<7> The method for measuring a urea concentration according to <6>, wherein the urea concentration in the spent dialysate during hemodialysis can be monitored in real time.
<8> An apparatus for measuring a urea concentration, the apparatus including:
   a second-reagent adding unit configured to add a second reagent including a xanthene dye or a rare earth fluorescent complex to a sample solution including urea to be mixed together to form a mixed solution;
   a first-reagent adding unit configured to add a first reagent including a hypohalite ion to the mixed solution to be allowed to undergo a reaction in a reaction cell; and a measuring unit configured to measure light intensity of chemiluminescence produced through the reaction to determine the urea concentration.
<9> The apparatus for measuring a urea concentration according to <8>, wherein the apparatus includes a first- and second-reagents adding unit configured to add the second reagent to the sample solution including urea to be mixed together to form a mixed solution, and to add the first reagent to the mixed solution to be allowed to undergo a reaction in a reaction cell.

## Claims

1. A method for measuring a urea concentration, the method comprising:
adding a first reagent and a second reagent to a sample solution comprising urea to be allowed to undergo a reaction, and measuring light intensity of chemiluminescence produced through the reaction to determine the urea concentration, the first reagent comprising a hypohalite ion, the second reagent comprising a xanthene dye or a rare earth fluorescent complex.

2. The method for measuring a urea concentration according to claim 1,
wherein the second reagent is added to and mixed with the sample solution comprising urea to form a mixed solution, and the first reagent is mixed with the mixed solution to be allowed to undergo the reaction.

3. The method for measuring a urea concentration according to claim 1 or 2,
wherein the xanthene dye comprises at least one selected from the group consisting of Rhodamine B, fluorescein, dibromofluorescein, and dichlorofluorescein.

4. The method for measuring a urea concentration according to any one of claims 1 to 3,
wherein the hypohalite ion comprises at least one selected from the group consisting of a hypofluorite ion (FO-), a hypochlorite ion (ClO⁻), a hypobromite ion (BrO⁻), and a hypoiodite ion (IO⁻).

5. The method for measuring a urea concentration according to claim 4,
wherein the hypohalite ion comprises the hypobromite ion (BrO⁻).

6. The method for measuring a urea concentration according to any one of claims 1 to 5,
wherein the sample solution comprising urea comprises spent dialysate during hemodialysis.

7. The method for measuring a urea concentration according to claim 6,
wherein the urea concentration in the spent dialysate during hemodialysis can be monitored in real time.

8. An apparatus for measuring a urea concentration, the apparatus comprising:
a second-reagent adding unit configured to add a second reagent comprising a xanthene dye or a rare earth fluorescent complex to a sample solution comprising urea to be mixed together to form a mixed solution;
a first-reagent adding unit configured to add a first reagent comprising a hypohalite ion to the mixed solution to be allowed to undergo a reaction in a reaction cell; and
a measuring unit configured to measure light intensity of chemiluminescence produced through the reaction to determine the urea concentration.

9. The apparatus for measuring a urea concentration according to claim 8,
wherein the apparatus comprises a first- and second-reagents adding unit configured to add the second reagent to the sample solution comprising urea to be mixed together to form a mixed solution, and to add the first reagent to the mixed solution to be allowed to undergo a reaction in a reaction cell.
